# EUROPEAN PATENT APPLICATION

(11) **EP 4 523 695 A1**
(43) Date of publication of application: **19.03.2025**
(21) Application number: 23803604.0
(22) Date of filing: 11.05.2023
(51) Int. Cl.: A61K 35/742, A61P 37/02, C12M 1/00, C12N 1/20, C12N 15/31

(54) **LIQUID CULTURE MEDIUM FOR IMPROVING IMMUNITY AND PROMOTING NORMAL IMMUNE FORMATION, AND LIQUID CULTURE MEDIUM GENERATOR**

(30) Priority: 12.05.2022 JP 2022078776
(71) Applicant: Naturesupport Co., Ltd., Takayama-shi, Gifu 506-0059 (JP)
(72) Inventor: KUBO Kensuke, Takayama-shi, Gifu 506-0059 (JP)
(74) Representative: Henkel & Partner mbB
(86) International application number: PCT/JP2023/017722
(87) International publication number: WO 2023/219130

(57) **Abstract**

[Object] Provided is a culture solution for promoting immune improvement and normal immune formation that promote healthy immune formation of humans and cure allergic diseases such as atopic dermatitis, and a culture solution generator capable of easily generating the culture solution.

[Solving Means] A culture solution generator 1 generating a culture solution for promoting immune improvement and normal immune formation, the culture solution obtained by suspending 300 g or more of Bacillus fermented powder in 150 liters of water, maintaining a temperature of 30 to 45°C, and supplying oxygen with an oxygen saturation of 70% or more for fermentation.

## Description

### TECHNICAL FIELD

The present invention relates to a culture solution for immune improvement and normal immune formation for promoting healthy immune formation of humans and curing allergic diseases such as atopic dermatitis, and a culture solution generator.

### BACKGROUND ART

Conventionally, for the treatment of dermatitis, a private therapy has been performed by some devotees, in which a fermented powder (refer to, for example, Patent Document 1) of Makomo, a type of wild rice native to rivers in Japan is suspended in hot water of a bath and bathing is performed for a long period of time without changing the bath water.

### PRIOR ART DOCUMENT

### PATENT DOCUMENT

Patent Document 1: JP H8-131158 A

### SUMMARY OF THE INVENTION

### PROBLEMS TO BE SOLVED BY THE INVENTION

However, such a private therapy is problematic in that the effect is different and the scientific mechanism is not clarified, the effect is unstable, and the side effect is unknown. Further, in the conventional therapy, the temperature of the culture solution and the oxygen supply are not appropriately controlled, arising such a problem that a culture solution suitable for treatment cannot be generated and maintained, for example, unnecessary bacteria and anaerobes propagate to generate unpleasant odor, effective bacteria decrease to reduce the effect, and harmful pathogenic bacteria propagate.

The present invention has been made in view of such problems, and an object of the present invention is to provide a culture solution for promoting immune improvement and normal immune formation that promote healthy immune formation of humans and cure allergic diseases such as atopic dermatitis, and a culture solution generator capable of easily generating the culture solution.

### MEANS FOR SOLVING THE PROBLEM

The present invention has been made to solve at least a part of the above-described problems, and can be realized as the following application examples. Note that reference signs in parentheses, supplementary explanation, and the like in this column indicate correspondence with embodiments to be described later in order to help understanding of the present invention, and do not limit the present invention at all.

### [Application Example 1]

The invention described in Application Example 1 is
a culture solution for promoting immune improvement and normal immune formation, the culture solution obtained by suspending 300 g or more of the Bacillus fermented powder in 150 liters of water, maintaining a temperature of 30 to 45°C, and supplying oxygen with an oxygen saturation of 70% or more for fermentation.

Suspending 300 g or more of the bacillus fermented powder in 150 liters of water and maintaining the temperature at 30 to 45°C causes new fermentation, and provides a culture solution having various bacteria that stimulate immunity of a human body and improve immunity. At the same time, supplying oxygen with an oxygen saturation of 70% or more and maintaining aerobic causes effective bacteria to be efficiently cultured, and allows the generation of harmful bacteria to be suppressed.

The bathing therapy using such a culture solution (hereinafter, referred to as biological spa care (BSC)) improves the immunity of the human body by various bacteria in the culture solution, and allows allergic diseases such as atopic dermatitis to be suppressed. In addition, applying BSC to the early childhood when basic immunity is formed promotes the development of the skin immunity of infants and allows to prevent the onset of allergic diseases such as atopic dermatitis.

Further, drinking such a culture solution can be expected to activate intestinal immunity in children to adults, and the immune improving effect thereof can be expected to exert the effect in the treatment of cancer. In particular, a synergistic effect can be expected in combination with existing surgery, radiation therapy, anticancer drug therapy, and immunotherapy.

### [Application Example 2]

The culture solution for promoting immune improvement and normal immune formation described in Application Example 2 has,
in the culture solution for promoting immune improvement and normal immune formation according to Application Example 1,
a bacterial concentration of 1 × 10⁹ copies/ml or more in real-time PCR;
contains 180 or more types of bacteria by NSG analysis of the base length of V3 to V4 region 430bp of 16S rDNA; and
contains ectobacillus funiculus as a dominant bacterium.

In such a culture solution, suspending the bacillus fermented powder in water, supplying oxygen at an appropriate temperature, and culturing allow a large number of living bacteria having high immune activity to be propagated, and particularly, Ectobacillus funiculus, which is a spore fungus belonging to the genus Ectobacillus and is not detected in a powder state, is propagated as the most preferred bacterium.

Bacilli bacteria such as Ectobacillus funiculus have a function of decomposing plants on the ground surface to form surface soil, and have been in contact with human skin from ancient times and deeply involved in the evolution of skin immunity. Many of these bacteria form spores and, as gram-positive bacilli, are intricately involved in stimulating skin immunity. Considering that Ectobacillus funiculus is the most preferred bacteria in the culture solution, it is highly likely that this bacterium stimulates the skin's immune system most effectively, improves the human body's immune system, and enhances the therapeutic effect of atopic diseases such as atopic dermatitis.

### [Application Example 3]

A culture solution generator for promoting immune improvement and normal immune formation according to Application Example 3 is
a culture solution generator for generating a culture solution for promoting immune improvement and normal immune formation according to Application Example 1 or Application Example 2, the culture solution generator including:
a heater unit (30) configured to heat the culture solution;
a stirring unit (20) configured to stir the culture solution; and
a control unit (40) configured to increase a temperature of the culture solution by the heater unit (30) to maintain at a desired temperature.

For the immune improvement and normal immune formation effect by the culture solution using the Bacillus fermented powder, a similar immune improvement effect has been confirmed to be obtained not only by bathing like BSC but also by using as a lotion to be applied to the skin.

Such a culture solution generator (1) is compact and easy to use, and can easily set appropriate culture conditions for immune improvement and normal immune formation of the skin, and can easily generate a culture solution to be applied to the skin, particularly as a lotion, at home.

### BRIEF DESCRIPTION OF THE DRAWINGS

FIG. 1 is a diagram showing the number of bacteria in a culture solution according to the present invention.
FIG. 2 is a diagram showing classification results of microbiota in a culture solution according to the present invention.
FIG. 3 is a diagram showing a therapeutic effect by BSC.
FIG. 4 is a diagram showing changes in immunity by BSC.
FIG. 5 is a diagram showing measured values of changes in serum cytokines by BSC.
FIG. 6 is a graph showing measured values of changes in serum cytokines by BSC.
FIG. 7 is a diagram showing measured values of peripheral blood mRNA before and after treatment by BSC.
FIG. 8 is a graph showing measured values of peripheral blood mRNA before and after treatment by BSC.
FIG. 9 is a diagram showing treatment results in case 1.
FIG. 10 is a diagram showing treatment results in case 2.
FIG. 11 is a diagram showing treatment results in case 3.
FIG. 12 is a diagram showing treatment results in case 4.
FIG. 13 is a schematic external diagram of a culture solution generator.
FIG. 14 is a configuration diagram showing a schematic configuration of a control unit.
FIG. 15 is a diagram showing a heating effect on fungus in a culture solution in a culture solution generator.
FIG. 16 is a diagram showing a stirring effect in the culture solution generator.

### MODE FOR CARRYING OUT THE INVENTION

Hereinafter, embodiments to which the present invention is applied will be described. The embodiments of the present invention are not limited to the following embodiments at all, and can take various forms as long as they fall within the technical scope of the present invention.

In the culture solution according to the present invention, the fermented powder of Makomo wild rice is suspended as a bacillus fermented powder in water, and is subjected to temperature control, oxygen supply, and stirring to cause new fermentation. When the conditions are satisfied, aerobic propagation of original Bacillus bacteria occurs without causing anaerobic decay, and metabolic decomposition of organic substances in the bacillus fermented powder occurs.

### (Production of Bacillus fermented powder)

It is possible to produce a fermented powder containing a large amount of Bacillus bacteria by using a material derived from a gramineous plant such as Makomo wild rice, reed, Kaya, or rice straw, or a plant such as bran or cereals. In particular, the plant (raffinate plant) on the water side in a natural state is suitable as a material because spores of effective Bacillus bacteria such as Bacillus subtilis are attached thereto.

Herein, Bacillus bacteria will be described. The Bacillus bacteria are heterotrophic bacteria that widely inhabit in soil and decompose dead plants, and Bacillus subtilis natto with low pathogenicity is also one of them. Many of the Bacillus bacteria form durable spores and enter a dormant state when the growth environment deteriorates. The Bacillus bacteria include obligate aerobic bacteria that always require oxygen, such as Bacillus subtillis, Bacillus megaterium, and Bacillus lentus, and facultative aerobic bacteria that perform anaerobic metabolism in environments where oxygen is not available, such as Bacillus circulans, Bacillus licheniformis, and Bacillus cereus.

Performing fermentation control such as oxygen and temperature control allows to produce Bacillus fermented powder containing a large amount (1×10⁸ CFU/g or more) of effective Bacillus spores. The particle size of the powder is desirably 100 µm or less in diameter in order to increase suspended particles and reduce precipitation on the bottom. Bacillus fermented powder requires sufficient fermentation to reduce substances that may act as antigens on the skin, such as proteins, as well as harmful substances and harmful microorganisms.

### (Production of culture solution)

For the culture solution, 300 g or more of Bacillus fermented powder such as a fermented powder of Makomo wild rice is suspended in 150 L of water, the temperature is controlled at 30 to 45°C, oxygen is supplied with an oxygen saturation of 70% or more while stirring, and new fermentation is performed for several days. When the conditions are satisfied, aerobic propagation of original Bacillus bacteria occurs without causing anaerobic decay, and metabolic decomposition of organic substances in the bacillus fermented powder occurs. Once the conditions are met, the culture solution has a bacterial concentration of 1 × 10⁹ copies/ml or more in real-time PCR.

In the vicinity of the bottom of still water with less oxygen, anaerobic metabolism by facultative aerobic bacteria and anaerobic bacteria is performed, and hydrogen sulfide (H₂S) and methane (CH₄) which are harmful to the human body and also causes malodor, propionic acid and normal butyric acid of mercaptan lower fatty acid, and the like are formed. These malodorous substances are not only uncomfortable but also harmful to the human body and cause dermatitis to be worsened.

In addition, depending on the optimum growth temperature, bacteria are classified into three groups: 10 to 20°C: low-temperature bacteria; 30 to 40°C: medium-temperature bacteria; and 50 to 60°C: high-temperature bacteria. Herein, most of pathogenic bacteria are included in medium-temperature bacteria. On the other hand, many Bacillus bacteria, which are soil bacteria, can grow even at high temperatures and have a spore-forming ability, and once spores are formed, they cannot be inactivated even at 100°C.

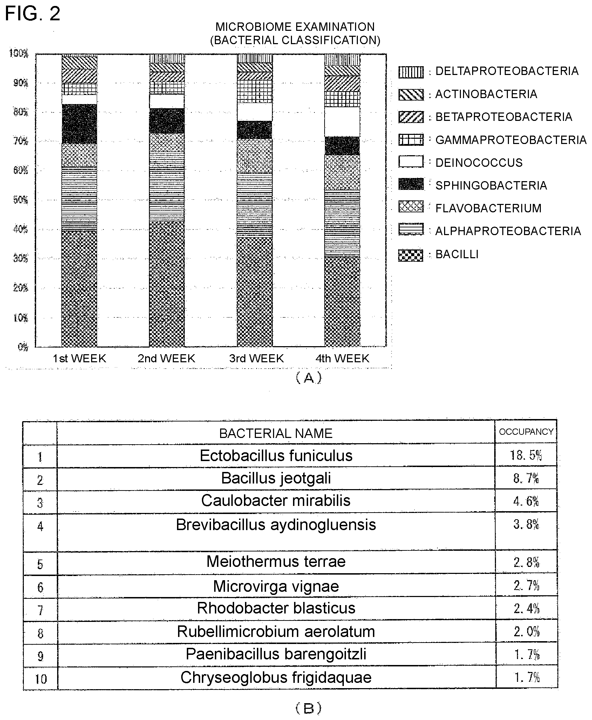

Therefore, in order to maintain the number of highly active Bacillus bacteria in the culture solution and reduce harmful substances produced from the metabolism of the Bacillus fermented powder and fats and proteins excreted from the human body, it is necessary to supply oxygen to maintain aerobic water quality conditions, constantly stir suspended particles that are likely to precipitate, stir to improve anaerobic conditions in the lower bath to promote volatilization of harmful substances, and control the temperature capable of safely maintaining the culture temperature for 24 hours. FIG. 2(A) shows the weekly reduction over time of the Bacilli bacteria in the bath water.

In the BSC, this culture solution is used exclusively for one bathing person with dermatitis. In addition, the therapeutic effect of dermatitis is reduced from around 3 weeks later, and thus this culture solution is replaced at intervals of 3 to 4 weeks. It is considered that the cause is mainly a change in bacterial flora and a decrease in clinical effect over time of bacteria belonging to the class Bacillus.

### (Confirmation of number of bacteria contained in culture solution)

Using Themal Cycler Dice Real Time System III and TB Green Fast qPCR Mix (Takara Bio Inc.), plasmid DNA containing a target region was requested to Takara Bio Inc. for each of the following primer sets and prepared.

Then, a standard curve was created using the prepared plasmid DNA, and the copy number was quantified. Primers are used as all bacteria:
Eub338F: ACTCCTACGGGAGGCAGCAG
Eub518R: ATTACCGCGGCTGCTGG,
Fungi
ITS1F: CTTGGTATTTAGAGGAGTAA
ITS4R: TCCTCCGCTTATTGATATGC,
spore-forming bacteria
spoOa655F: GGHGTDCCNGCNCATATHAA
spoOa923R: GCDATGAAYTCDGAGTTNGTNGG.

The same conditions as those for suspending 100 g, 200 g, 300 g, 400 g, 500 g, and 600 g of Bacillus fermented powder manufactured by Naturesupport in 150 L of bath water and aerobically culturing at 40°C were made in a beaker, DNA was extracted from the culture solution cultured for 5 days, and the total number of bacteria was measured by real-time PCR at n = 3. An average value was obtained except for outliers, and a logarithmic graph was obtained.

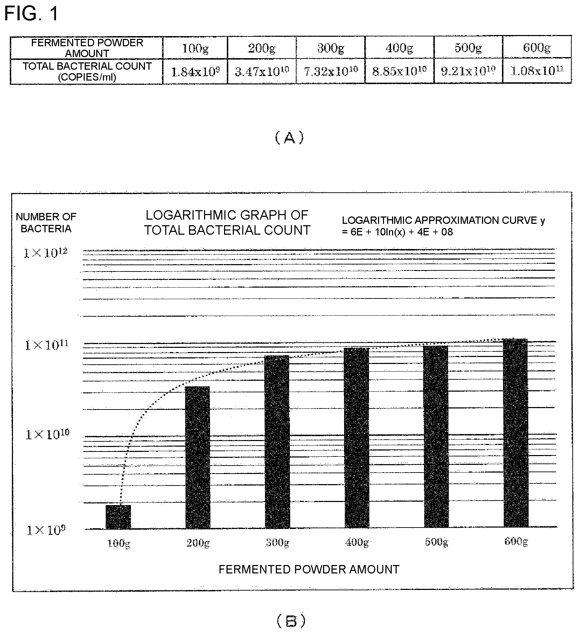

FIG. 1 shows the measurement results of the number of bacteria with respect to the amount of the Bacillus fermented powder. FIG. 1(A) shows a measurement result of the number of bacteria, and FIG. 1(B) shows a logarithmic graph of the measurement result. Bacteria (total number of bacteria) of 1 × 10⁹ to 1 × 10¹¹ copies/ml were observed in the culture solution by real-time PCR. The number of bacteria reaches almost the upper limit with a dose of 300 to 400 g of the Bacillus fermented powder in 150 L of bath water.

Considering the increase in cost and sediment, anaerobic metabolism in sediment, and biofilm increase, 300 to 400 g of bath agent is suitable, and there will be 1 × 10⁹ copies/ml or more of bacteria in the culture solution.

### (Classification result of microbiota existing in culture solution according to the present invention)

Then, a meta 16SrRNA gene analysis was performed on the microbiota in a total of 12 samples of bath water 1 week, 2 weeks, 3 weeks, and 4 weeks after the start of culture with a high treatment effect in 3 baths.

The test was performed using Miseq Reagent Kit v3 (600 cycles), which is an NSG from Illumina, Inc., with a primer, 341f: CCTACGGGAGGCAGCAG R806: GGACTACHVGGGTWTCTAAT, at a base length of V3 to V4 region 430bp of bacterial 16SrDNA. Analysis by class was performed at a homology rate of 80% using database RDP. Analysis by species was performed at a homology rate of 97% using a database DB-BA ver. 13.0 of TechnoSuruga Laboratory Co., Ltd.

In the bacterial species classification, about 40% of about 35000 leads on average of 1 specimen could be classified, and 186 to 271 species and 233 species on average were classified. At the class level, the top 9 species accounted for 89%. FIG. 2(A) shows the classification results at the class level.

A characteristic point compared to Microbiome such as human body, soil, and activated sludge is that there are many Bacilli bacteria which are gram-positive spore bacteria, and Bacillus bacteria exist as a dominant bacterium in a culture solution, and occupy 38% at the maximum.

The bacterial flora is greatly changed by being cultured in the bath water after the bath agent powder of the bath water is added. In the genus classification, 40% is replaced, and in the species classification, 70% to 80% is replaced. Thereafter, waste products from the human body are also added, and the bacterial flora changes.

The occupancy of spore bacteria in the culture solution is about 25 to 40%. The gram-positive spores of the Bacilli are considered to be clinically deeply involved in immunostimulation of the skin, but decrease with the progress. The therapeutic effect of the bath water also decreases, and thus it is necessary to replace the bath water in three to four weeks.

The top 10 species for which the species classification was possible are shown in FIG. 2(B). The top 5 species account for 38.4%, and the top 10 species account for 48.9%. In the culture solution, Ectobacillus funiculus was present as the most dominant bacterium and occupied 18.5%.

In the microbiome test of the fermented powder by NGS, Ectobacillus funiculus is not observed at all, but it seems that those slightly attached to the raffinate plant propagate under the environment of water and become the most dominant bacteria.

The nucleotide sequence of Ectobacillus funiculus at a base length of V3 to V4 region 430bp of 16S rDNA is as follows:

“TAGGGAATCTTCGGCAATGGGCGAAAGCCTGACCGAGCAACGCCGCGTGAGC
GATGAAGGCCTTCGGGTCGTAAAGCTCTGTTGTTAAGGAAGAACAAGTACGAGAGTAAC
TGCTCGTACCTTGACGGTACTTAACGAGAAAGCCACGGCTAACTACGTGCCAGCAGCCG
CGGTAATACGTAGGTGGCGAGCGTTATCCGGAATTATTGGGCGTAAAGCGCGCAGGC
GGCTTCTTAAGTCTGATGTGAAAGCCCACGGCTCAACCGTGGAGGGTCATTGGAAACTG
GGAAGCTTGAGTGCAGAAGAGGAAAGCGGAATTCCACGGGTAGCGGTGAAATGCGTAGA
GATGTGGAGGAACACCAGTGGCGAAGGCGGCTTTCTGGTCTGTAACTGACGCTGAGGCG
CGAAAGCGTGGGGAGCAAACAGGAT”.

As a result, in order to stimulate the skin immunity and normalize the immunity, it is considered that it is necessary to include Bacillus jeotgali, Caulobacter mirabilis, Brevibacillus aydinogluensis, and Meiothermus terrae as the dominant bacteria in the culture solution with Ectobacillus funiculus as the most dominant bacteria.

Gram-positive bacteria mainly induce Th1 immunity, and gram-negative bacteria can induce Treg immune tolerance. In the intestinal tract in mice, it is widely known that among many bacterial groups, segment filamentous bacteria that are gram-positive, anaerobic, spore-forming bacteria from the phylum Firmicutes and the family Clostridiaceae strongly induce immunity of IgA, Th1, and TH17.

From many case experiences, it has been observed that the clinical effect is reduced by temporal reduction of the Bacilli bacteria of the phylum Firmicutes in bath water. Therefore, it is considered that Ectobacillus funiculus, which is the most dominant bacterium of the Bacilli, is most involved in the immune effect.

### (Study results for confirmation of effects of immune improvement and normal immune formation by bathing therapy using culture solution)

In order to confirm the immune improvement and normal immune formation effect by BSC, studies were performed on the immune improvement effect on adult severe atopic dermatitis patients and the effect as a basic immunogenic vaccine in neonates and infants.

### (Confirmation of effect of immune improvement in adult severe atopic dermatitis patient)

Patients were divided into the following three groups:

### (1) Group 1

The therapeutic effect after BSC at an average of 76 days of hospitalization was measured for 55 adult severe atopic dermatitis patients. The change in Th1/Th2 by the flow cytometer was measured in 29 of them.

### (2) Group 2

Changes in blood cytokines before and after BSC were measured in 20 hospitalized patients different from Group 1.

### (3) Group 3

Changes in mRNA expression of white blood cells before and after BSC were measured in 20 hospitalized patients different from Group 1 and Group 2.

### (Treatment results in Group 1)

55 patients (average age 34.7 years, 24 males and 31 females) of severe patients aged 18 years or older who were diagnosed as atopic dermatitis according to the diagnostic criteria of Japanese Dermatological Association, had a skin rash accompanied by strong inflammation at 10% or more of the body surface as the severity, and had 17 or more in the severity classification of POEM (0 to 28) were targeted.

For 150 L of culture bath water, about 400 g of the fermented powder of Makomo wild rice as a Bacillus fermented powder was suspended, and oxygen with an oxygen saturation of 70% or more was supplied and stirred at a temperature of 35 to 42°C for 3 to 7 days in a bath using a dedicated culture solution circulating apparatus that was always operated for 24 hours to control the temperature and supply oxygen.

All patients took a bath for 2 to 4 hours per day for 4 to 20 weeks (average 75.7 days) using a dedicated bathroom. The bath water was used every day without replacement, and the bath water was replaced every 3 to 4 weeks or when itching was worsened or the improvement speed was reduced. As the Bacillus fermented powder and the dedicated circulating apparatus, those sold by Naturesupport were used.

A large change in bacterial flora is completed in about four days from the bath powder suspension. After the start of bathing, bacterial flora that decomposes desquamation and secretion of human skin is developed. In addition, the number of protozo that prey on bacteria also increases, but decreases in about two weeks. The bacterial flora always changes, and the skin immunity is repeatedly stimulated.

For the determination of the therapeutic effect, subjective symptoms of the patient and blood markers were used as indices. As subjective symptoms, a questionnaire POEM: Patient-Oriented Eczema Measurement (POEM0-28) was used, which was written by the patient himself.

POEM is an internationally recognized scale of severity of atopic dermatitis together with EASI, and the severity classification is 0 to 2 (clear/almost clear); 3 to 7 (mild); 8 to 16 (moderate); 17 to 24 (severe); and 25 to 28 (very severe). Compared with the EASI strongly influenced by the subjectivity of the observer, POEM focusing on the subjective symptom of the patient has higher objectivity.

As blood markers, three markers of serum TARC, total IgE, and % eosinophils were adopted to have objectivity. For the normal values, TARC has 450 pg/ml or less, total IgE has 170IU/ml or less, and % eosinophils has 7% or less. The test was performed at BML. Co. JP as a clinical test. In particular, it is internationally recognized that TARC (Thymus and activation-regulated chemokine) correlates with the severity of atopic dermatitis, and 2000 or more indicates moderate, and 5000 or more indicates approximately severe.

Comparison between baseline at admission and numerical values obtained at discharge was performed using t-test for standard distribution data and Wilcoxon signed rank sum test, which is a non-parametric test, for discrete data. Mann-Whitney test was used to test selection from the population. Data are shown as mean ± standard error (SE).
p ≤ 0.05 was regarded as statistically significant, and denoted as *p ≤ 0.05 and **p ≤ 0.01.

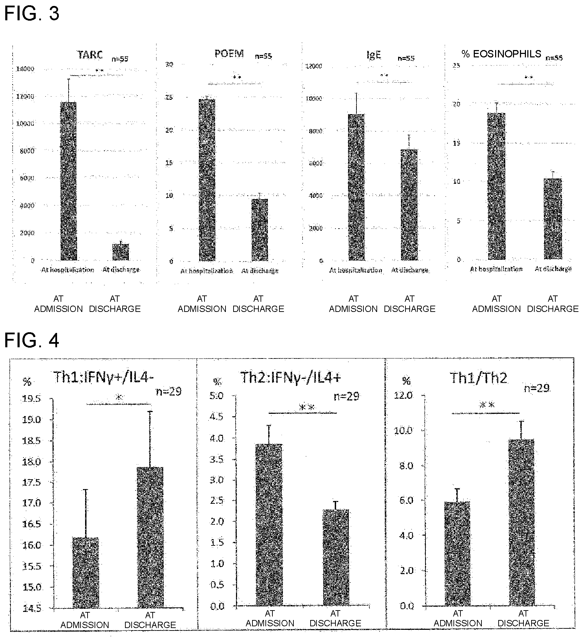

As shown in FIG. 3, the mean POEM score decreased from 24.7 ± 0.4 at admission to 9.5 ± 0.8 (n = 55, P < 0.001) at discharge by 61.8%. The severity was reduced from most severe to moderate.

FIG. 3 shows a measurement result of each index at the time of admission and discharge. Mean TARC levels were all reduced, with a significant reduction of 89.6% from 11562.5 ± 1701.8 pg/ml at admission to 1197.3 ± 194.3 pg/ml (n = 55, P < 0.001) at discharge. TARC improved by 72.7% at 1 month after admission, and improvement from early stage was observed.
% eosinophils decreased from 18.9 ± 1.2% to 10.4 ± 1.0% (n = 55, P < 0.001) and total IgE also decreased from 9046.9 ± 1288.1 IU/ml to 6863.7 ± 899.0 IU/ml (n=55, P < 0.001).

At the time of discharge, all patients had improved skin rash accompanied by strong inflammation, had only mild inflammation or dry skin, or had normal skin.

Next, to elucidate the mechanism behind the clear effectiveness of BSC, the inventor investigated the changes in patients' immunity. Many studies have shown in the past that atopic dermatitis patients are clearly found to have lower peripheral blood T-lymphocyte subtype CD4+/IFNγ+ and higher CD4+/IL-4+ compared to healthy people. It is well known as a Th1/Th2 imbalance in atopic dermatitis.

Herein, Th1 and Th2 will be described. There are two types of immunity: innate immunity (cellular immunity) and acquired immunity (humoral immunity). The T cell lymphocytes of acquired immunity are largely divided into two lineages, Th1 and Th2. Th1 functions in conjunction with innate immunity (cellular immunity), and Th2 functions in conjunction with acquired immunity (humoral immunity). Therefore, Th1/Th2 ratio, which is the activity ratio between Th1 and Th2, is an easy-to-understand index for grasping the immune constitution. In allergy, Th2 activity is high, and the Th1/Th2 ratio is low. TARC, which is a biomarker indicating the severity of atopic dermatitis, is also one of cytokines involved in Th2 immunity.

Mammals have a mechanism called immune tolerance in which Th1 immunity, which is cytotoxic immunity, is suppressed to maintain pregnancy so that mother and child with different genes do not damage each other as an immune foreign substance during pregnancy. Therefore, at the time of birth, Th1 immunity of the newborn infant is suppressed, and Th2 immunity is in an excessive state. The birth is accompanied with a so-called allergic diathesis.

The inventor measured CD4 (IFNγ+/IL4-) (referred to as Th1 for convenience) and CD4 (IFNγ-/IL4+) (referred to as Th2 for convenience) in peripheral blood using flow cytometry analysis in 29 out of 55 patients selected for convenience. The measured patients were selected for convenience, but the values of POEM score, TARC, and % eosinophils were not different from the population in the Mann-Whitney test.

Changes in immunity caused by BSC will be described with reference to FIG. 4.

In the atopic dermatitis patient group in this case, Th1 was 16.2 ± 1.2% and Th2 was 3.9 ± 0.4% at the time of admission, Th1 was lower than the average of 21.3% of healthy subjects, and Th2 was higher than the average of 2.7% of healthy subjects, indicating an atopic specific immune state having Th2 dominance. (The numerical value of healthy subjects is based on "Study of 53 Healthy Persons by Tanaka et al.: Analysis of Kinetics of Helper T Cell Subsets (Th1, Th2) in Healthy Adult Peripheral Blood Using Flow Cytometer, Clinical Pathology, 1998.12")
However, the imbalance of Th greatly changes before and after BSC, and
Th1 (IFNγ+/IL4-) was 16.2 ± 1.2% at admission, 17.9 ± 1.3% (10.5% improvement) at discharge, P < 0.05,
Th2 (IFNγ-/IL4+) was 3.9 ± 0.4% at admission and 2.3 ± 0.2% (40.9% improvement) at discharge, P < 0.01, indicating clear improvement.

### (Treatment results in Group 2)

Then, serum cytokine levels were measured for 20 atopic dermatitis patients (age average 38.4 years, 7 males, average number of days of admission 80 days) before and after admission and 17 control healthy subjects (age average 35.9 years, 7 males) by a multiplex suspension array.

Using Bio-Plex Pro Human Cytokine 17-Plex Panel (F-M50-00031YV) of Bio-Rad Laboratories, Inc., 17 cytokines IL-1β, IL-2, IL-4, IL-5, IL-6, IL-7, IL-8, IL-10, IL-12 (p70), IL-13, IL-17, G-CSF, GM-CSF, IFN-gamma, MCP-1 (MCAF), MIP-1β, and TNF-α were measured, and by ELISA, TGF-β1 (R & D) was measured at an assay range of 1.7 to 15.4 pg/mL and TSLP (Thermo Fisher Scientific) at an assay range of 3.28 to 800 pg/mL. The specimen was measured at n = 2, and the standard was measured at n = 3.

The measured value includes a value calculated from the regression equation of the standard curve even if the value is outside the standard curve range. A value less than the detection limit was set to a half value of the minimum detection value. For statistics, Wilcoxon signed rank sum test was used for comparison between the time of admission and the time of discharge, and Mann-Whitney test was used for comparison between the time of admission and a healthy control person. p ≤ 0.05 was considered statistically significant. In addition, TARC and % eosinophils are numerical values obtained by BML. Co. JP as a clinical test.

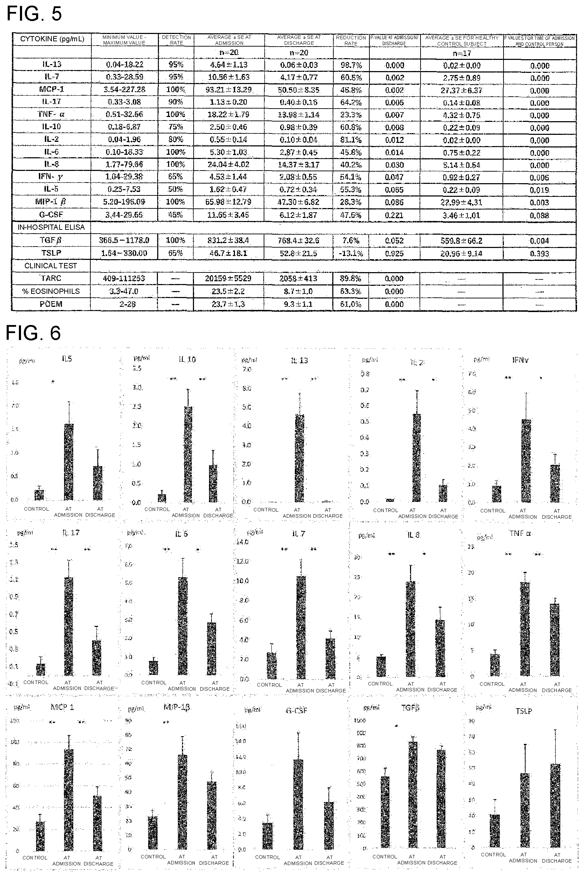

Changes in serum cytokines before and after BSC will be described with reference to FIGS. 5 and 6. FIG. 5 shows measured values of each cytokine, and FIG. 6 shows a graph of measured values of 15 items that could be measured among the 19 items of the test. Among the 19 test items, 10 out of the 15 measurable items, IL-13, IL-2, IL-7, IL-17, IL-10, IL-6, IL-8, IFN-γ, TNF-α, and MCP-1 showed a statistically significant decrease before and after BSC. POEM improved from 23.7 ± 1.3 to 9.3 ± 1.1 by 61%, TARC improved from 20159 ± 5529 to 2058 ± 413 by 89.8%, and % eosinophils improved from 23.5 ± 2.2 to 8.7 ± 1.0 by 63.3%.

Hypersecretion in peripheral blood of all cytokines other than TSLPs involved in inflammation, such as various cytokine groups Th1 (IL2, IFN-γ), Th2 (IL5, IL10, IL13), Th17 (IL17), and the pro-inflammatory cytokines (IL6, IL7, IL8, TNF-α, MCP-1) in severe atopic dermatitis, was suppressed by the treatment.

When IFN-γ/IL13 was used as an index as a Th1/Th2 ratio, by BSC, the ratio increased 24.6 times (P = 0.0001) and IL2/IL13 increased 22.2 times (P = 0.0002).

From this, it is considered that a large variety of immunostimulation contained in the culture solution act on the skin immunity to correct the immune abnormality such as allergy.

Recent findings of atopic dermatitis indicate that it is a complicated cytokine storm state in which Th17 and Th1 cytokines in a chronic phase are added to Th2 cytokine based inflammation in an acute phase, but immunostimulation by BSC controls complicated allergic inflammation while improving Th1/Th2 balance. In particular, the reduction rate of the typical Th2 cytokine IL13 was as high as 98.7%, which was characteristic.

With the advent of Dupilumab, which is an inhibitory monoclonal antibody formulation of IL4 and IL13 receptors, the significant impact of Th2 cytokines on atopic dermatitis is re-recognized, and Tralokinumab, which is an IL13 neutralizing antibody, also shows an effect. The marked reduction of IL13 by BSC also demonstrates the effect of BSC at cytokine levels, as IL13 has been shown to be deeply involved in inflammation and impaired barrier function, particularly in skin tissues.

From the above, it is considered that immunostimulation of the bacteria group to the skin by BSC controls the secretion of various inflammatory cytokines mainly including IL13 by some mechanism, and suppresses acute atopic dermatitis mainly including Th2 and Th17 cytokines and chronic atopic dermatitis to which Th1 cytokines are also added.

### (Treatment results in Group 3)

The expression of mRNA in peripheral blood leukocytes of 20 (12 males, average age of 32 years, average number of days of hospitalization of 81 days) patients from whom specimens were collected after BSC was tested at n = 3 using TaqMan Gene Expression Assays (Thermo Fisher Scientific) and compared with specimens of 20 healthy subjects (11 men, average age 29.6).

As a specimen, whole blood was collected with PAXgene Blood RNA Tubes (BD), cryopreserved at -20°C, RNA was separated and purified from whole blood cells by the procedure shown in PAXgene Blood RNA Kit (QIAGEN), and cDNA was synthesized using a reverse transcription reagent (Thermo Fisher Scientific).

The test item is IFNG, IL2, TBX21, IL4, IL13, IL10, GATA3, RORC, FOXP3, IL13RA1, and IL13RA2.

TARC, IgE, and % eosinophils were measured by BML. Co. JP as a clinical test, and the numerical values were reported. The detection rate was almost 100%, but the detection rate less than the detection limit was a half value of the minimum detection value. For statistics, Wilcoxon signed rank sum test was used for comparison between the time of admission and the time of discharge, and Mann-Whitney test was used for comparison between the time of admission and a healthy control person.

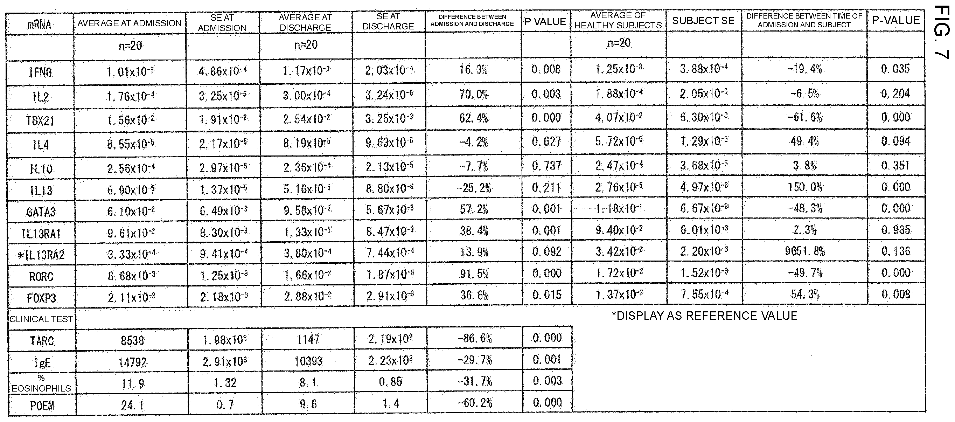

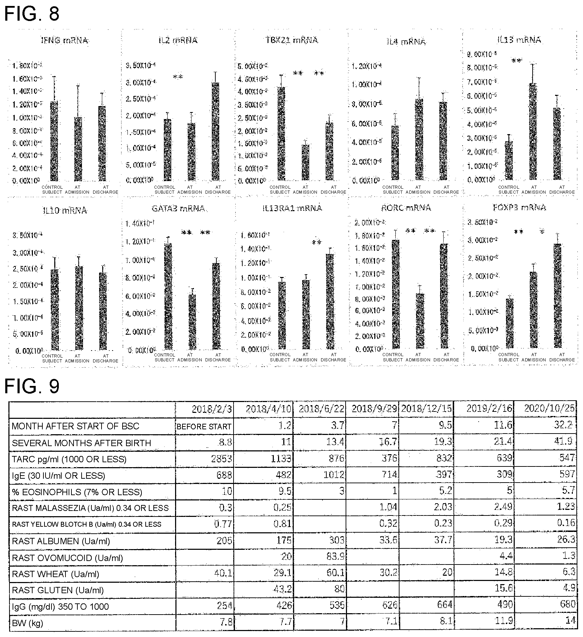

Based on FIG. 7 and FIG. 8, expression of peripheral blood mRNA before and after treatment will be described. FIG. 7 shows measured values of each mRNA, and FIG. 8 shows a graph of the measured value. In the patients before treatment, the expression of mRNA of IFNG and IL2, a Th1 cytokine, was lower than that in healthy subjects, and TBX21, a Th1 transcription factor, was significantly (P = 0.000) reduced.

Conversely, mRNA expression of IL4, IL10, and IL13, which are Th2 cytokines, was high. In particular, IL13 was significantly elevated by 150% (P = 0.009). A typical allergic pattern with so-called Th2 dominance was shown.

BSC significantly increased Th1 cytokine mRNA that was lower than that of healthy subjects. IFNG 16.3%(P = 0.008), IL2 70.0% (P = 0.003). In contrast, all of the Th2 cytokines IL4, IL10, and IL13, which were higher than those of the healthy subjects, decreased although not significantly.

The increase in Th1/Th2 by BSC was significantly confirmed, and the increase after treatment of IFNG/IL4 was 1.08 times (P = 0.044), IFNG/IL13 indicated 1.72 times (P = 0.004), and IL2/1L13 indicated 2.65 times (P = 0.001). TBX21, a transcription factor of Th1, Th2, Th17, and Treg, increased by 62.4% (P = 0.000), GATA3 by 57.2% (P = 0.001), RORC by 91.5% (P = 0.000), and FOXP3 by 36.6% (0.015), all with clear significant differences.

The increase rate of the Th17 transcription factor RORC is 91.5% (P = 0.000) and the increase rate of the Th2 transcription factor TBX21 is as high as 70% (P = 0.000), and it is considered that they reacted to a bacterial group in the bath water, and contrary to the decrease in secretion of serum cytokines, all of the transcription factors are increased, and may be involved in the suppression of atopic dermatitis.

Although the value of serum IL13 decreased by 98.7% in Group 2, the decrease in IL13mRNA in peripheral blood was only 25.2% (P = 0.211), and there was a deviation. In addition, although the mRNA expression of the Th17 transcription factor RORC was increased by 91.5%, the serum IL17 value was conversely decreased by 64.2% and there was a deviation. This is considered to be because the mRNA and the actual production of proteins such as cytokines have a moderate correlation but do not coincide with each other.

From the above, it is considered that immunostimulation of the skin by BSC has a clear influence on gene expression of peripheral blood lymphocytes flowing throughout the body.

### (Summary of confirmation test of immune improvement effect on adult severe atopic dermatitis patients)

The reduction rate of TARC by BSC was 89.6% in Group 1, 89.8% in Group 2, and 86.6% in Group 3, all of which showed clear improvement. This reduction rate shows an effect equivalent to that of Dupilumab (300 mg was subcutaneously injected every 2 weeks for 12 weeks with a TARC reduction rate of about 82.9%), which is recognized to be effective as a therapeutic agent for atopic dermatitis worldwide. Although BSC reduced serum IL13 by 98.7%, this fact also suggests that Dupilumab has an equivalent effect given that it is a block antibody against IL13 and IL4.

Although atopic dermatitis is based on the Th1/Th2 imbalance, it is considered to be a disease state in which innate immunity and acquired immunity Th1, Th2, Th17, Treg, ILC2, and the like are complicatedly involved.

It is considered that BSC plays a suppressive function of reducing almost all inflammatory cytokines while correcting the Th1/Th2 balance of systemic immunity through immunostimulation of the skin by bacterial groups.

### (Confirmation of effect as basal immunization vaccine in infancy)

Then, a treatment result of confirming the effect of the culture solution according to the present invention as an immunogenic vaccine will be described. It is said that basic immunization in infancy ends in a limited short period of infancy from neonatal period to about 2 years of age, and becomes basic immunity in which immunity formed in infancy lasts for a lifetime.

Therefore, using the culture solution according to the present invention, it can be expected to promote the original healthy immune formation of infants. In the following cases, immunostimulation with BSC was given to infants and toddlers 1 year of age or younger, who were considered to be undergoing basic immunization of the skin.

### (Case 1)

A 10 month old male. Moderate systemic atopic dermatitis from 4 months after birth. There was also food allergy, and particularly there was strong reaction to albumen.

A treatment result will be described with reference to FIG. 9.

The males underwent BSC for 30 min per day during a 40 day hospital stay. After discharge, BSC was continued at home for about 15 minutes. After 2 months from the start of BSC, TARC was reduced to half or less, and thereafter, TARC, % eosinophils, and IgE were smoothly reduced, and after 1 year, eczema on the skin disappeared, and the skin became completely normal.

In parallel with this, food allergies such as wheat, gluten, and albumen were also improved, and the value of a food allergic antibody was significantly reduced, and in particular, egg allergy was strong, and eczema occurred even in heated albumen (ovomucoid); however, the ovomucoid antibody was also rapidly reduced, and raw egg intake became possible after one year.

The IgG value indicating immune growth is higher than the IgE value, and normal immune growth is promoted. There is no allergic symptom such as allergic rhinitis, there is almost no viral infection such as common cold, and a healthy immune state in which the patient quickly recovers even if affected.

### (Case 2)

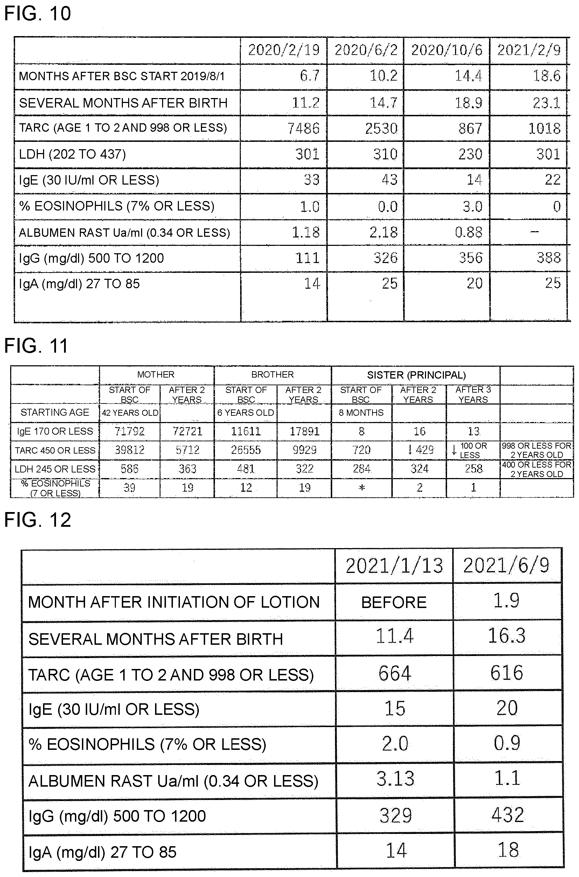

A female infant with atopic dermatitis with elevated TARC. Systemic atopic dermatitis has occurred from 3 months after birth. A treatment result will be described with reference to FIG. 10.

The bathing time was gradually increased starting from the extent of wetting the bath liquid with a towel, and after 4 months from the increase in the bathing time, the TARC decreased to 1/3 and the dermatitis was almost improved, and the TARC was normalized at 8 months. Thereafter, normal skin continued, and slight increase in TARC was observed, but IgE did not increase, and IgA and IgG antibodies increased, and thus, it is considered that immunogenesis has progressed.

### (Case 3)

A treatment result will be described with reference to FIG. 11.

In this case, the mother was a chronic and most severe atopic patient with IgE71797 and TARC39812, and the brother who started BSC at the age of 6 had also a most severe type of atopic dermatitis with IgE11611 and TARC26555.

However, the younger sister (case) who started BSC at 8 months after birth at the same time as the older brother had albumen allergy at the beginning, and had infant eczema, but which almost disappeared at 3 months after the start. The completely different point from the mother and the older brother is that, after 3 years, 100 or less of IgE13 and TARC do not show any allergic diathesis at all, and there is achieved the healthy body that has normal skin and hardly suffers from an infectious disease such as a cold.

Atopic dermatitis is also known as a disease with a high incidence in a family, and it is considered that the fact that genetic factors are similar to the home environment and diet affects atopic dermatitis.

In general, atopic dermatitis starts from infantile eczema developed by 1 year of age and gradually progresses, and thus it is understood how necessary it is to apply skin immunostimulation by BSC in the period of immunogenesis until 2 years of age. If the treatment with BSC is not performed, there is a high possibility that the same course as that of the older brother has been followed.

### (Case 4)

Infant eczema occurred on both cheeks at 1 month after birth. For this patient, instead of BSC, the culture solution was applied to the skin as a lotion. After 2 months from the start of applying the lotion, eczema on the cheek did not appear at all even without steroids. This case had a mild allergic diathesis, and had allergic infantile eczema in children who had normal IgE and TARC but started from food allergy.

Immunostimulation by the lotion cures dermatitis, suppresses an increase in IgE antibody, and reduces an allergic reaction to albumen, while promoting an increase in IgG and IgA antibodies showing normal immunogenesis.

This mechanism of immune development promotion is significantly important to prevent early infantile eczema from transitioning to atopic dermatitis. FIG. 12 shows measured results of various indexes.

### (Summary of test for confirming effect as basal immunization vaccine in infancy)

As described above, three cases in which BSC was applied to infants under 1 year of age and one example of lotion use have been presented, but in atopic dermatitis, the blood markers TARC and % eosinophils indicating allergy rapidly decrease to normal ranges.

The completely different feature from the other three examples of adult AD in which BSC was started within one year after birth and continued for one year or more is that there is no dermatitis at all and the skin has normal skin without even a moisturizer. In addition, allergic reactions such as allergic rhinitis and food allergy other than atopic dermatitis are hardly observed. No or significantly minor other influenza infections. a diathesis in which immunity is considered to be formed normally is acquired.

From the above, BSC is appropriate for severe atopic dermatitis and the like in children, and it is desirable to promote immunogenesis by applying a culture solution as a lotion to the skin in normal skin and infant eczema cases with a low allergic reaction. In addition, it is possible for a pregnant female to perform immunogenesis from a fetal stage by using BSC or lotion.

### (Other expected effects of BSC)

In recent years, a clinical effect of a PD-1 PD-L1 immune checkpoint inhibitor that inhibits one of cancer immune escape mechanisms represented by a molecular target therapeutic agent Nivolumab has been recognized. As an effect, it has been found that the immune suppression caused by cancer is corrected, the Th1/Th2 balance is greatly improved, and an antitumor effect is exhibited.

From the treatment results so far, it is considered that BSC corrects the Th1/Th2 balance by a mechanism different from that of immune checkpoint inhibitors, and thus the immune improvement effect of BSC is significantly likely to be exerted also in the treatment of cancer. In particular, a synergistic effect can be expected by using in combination with existing surgery, radiation, anticancer agents, and immunotherapy.

### (Effects expected from drinking culture solution according to the present invention)

Drinking the culture solution according to the present invention as a healthy beverage, immune improvement mainly by stimulation of intestinal immunity after school children is expected. In addition, it is expected that a pregnant female promotes development of immunity from a fetal stage by drinking. Further, it can also be used in mammals other than humans, and an effect of suppressing atopic dermatitis in pets, which has been increasing in recent years, is expected.

### [Configuration of culture solution generator 1]

In order to properly implement BSC, a system having a bath tank and a special circulation function is required, and in general households, it is difficult to prepare in terms of cost, and in order to receive BSC, it is necessary to go to a place where such equipment is well-equipped, and it takes cost and time.

Therefore, a culture solution generator 1 capable of easily generating the culture solution according to the present invention at home will be described. Using the culture solution produced using such a culture solution generator 1, it is possible to obtain an effect similar to that of BSC particularly by applying the culture solution to the skin as a lotion or drinking the culture solution.

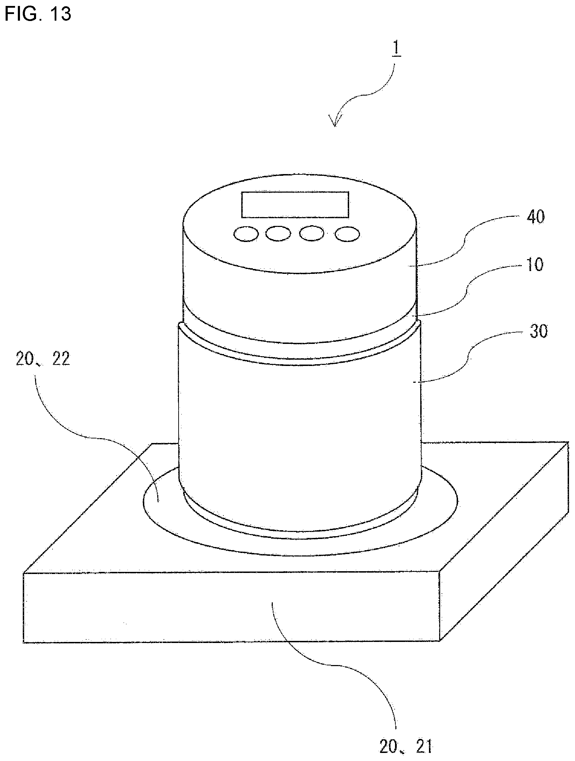

The configuration of the culture solution generator 1 will be described with reference to FIG. 13. FIG. 13 is a schematic external view of the culture solution generator 1. As shown in FIG. 8, the culture solution generator 1 includes a culture tank unit 10, a stirring unit 20, a heater unit 30, and a control unit 40.

The culture tank unit 10 is an unit for suspending and culturing water and the Bacillus fermented powder, and is formed in a cylindrical shape made of stainless steel, and one end portion thereof is closed by a flat plate made of stainless steel.

The stirring unit 20 uses a magnetic stirrer, and includes a stirring unit main body 21 and a stirrer (not shown). The stirrer is obtained by sealing a rod magnet with fluororesin or the like, is formed in a cocoon shape, an octagonal rod shape, or the like, and is installed inside the culture tank unit 10. Using the magnetic stirrer, the stirrer can be easily removed from the culture tank unit 10, and thus the inside of the culture tank unit 10 can be easily cleaned and sterilized, and the biofilm in which pathogenic bacteria such as legionella easily propagate can be easily removed in the culture tank unit 10.

The stirring unit main body 21 includes a top plate 22 made of ceramics or plastic, and a culture tank unit 10 is installed on the top plate 22. In addition, the stirring unit main body 21 includes a motor and a magnet (not illustrated), and rotates the magnet by the motor and rotates the stirrer installed inside the culture tank unit 10 by the generated magnetic force. The operation of the motor of the stirring unit main body 21 is controlled by a power switch provided in the stirring unit main body 21.

The heater unit 30 has a planar shape and is installed so as to wind around the outer periphery of the culture tank unit 10.

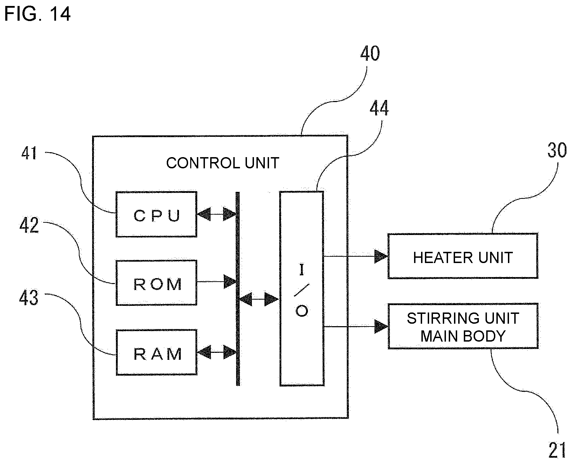

The control unit 40 has a columnar outer shape having the same diameter as the culture tank unit 10, and is installed on the culture tank unit 10. As shown in FIG. 14, an operation button and a display screen are provided, and a CPU41, a ROM42, a RAM43, and an I/O44 are provided inside the operation button and the display screen, and are connected to the operation button, the display screen, the stirring unit main body 21, and the heater unit 30 via the I/O44, and in response to an operation command by the operation button, the heating temperature by the heater unit 30, the heating time, and the motor drive of the stirring unit main body 21 are controlled.

The control unit 40 has the following functions (A) to (D).
(A) Turning on/off of the control unit 40
(B) Setting of heating temperature
(C) Setting of heating time
(D) Control of the heater unit 30 based on set temperature and time

### [Features of culture solution generator 1]

Using such a culture solution generator 1, the Bacillus fermented powder can be suspended in water, constantly controlled at a temperature of 30 to 45°C, supplied with oxygen having a saturation degree of 70% or more, allowing to easily generate a culture solution for improving the immunity of the skin.

In order to maintain an oxygen saturation of 70%, the culture solution is constantly stirred by a magnetic stirrer. This makes it possible to easily appropriately supply oxygen into the culture solution without requiring an oxygen supply device that requires a dedicated pump, a tube, or the like.

In addition, the culture solution cultured by the culture solution generator 1 can be warmed and sterilized at 65°C or more so that pathogenic bacteria such as legionella do not propagate in the biofilm inside the culture solution generator, and at the same time, replacement every week prevents the biofilm from remaining for a long period of time. In addition, making a cylindrical shape made of stainless steel without unevenness allows washing and treatment with a microbicide such as hypochlorous acid, monochloramine, or chlorine dioxide to be easily performed when the culture solution is replaced.

### (Fungus removing performance in culture solution generator 1)

The culture solution was stirred and cultured at 30 to 40°C in a flask, and one of the 40°C cultures was heated to 45°C for 10 hours a day to confirm a change in the number of eubacteria, spore fungi, and fungi. Fungus causes a strong allergic reaction to Malassezia and Candida which are a fungus of the skin in patients with atopic dermatitis, and thus it is necessary to eliminate from the culture solution.

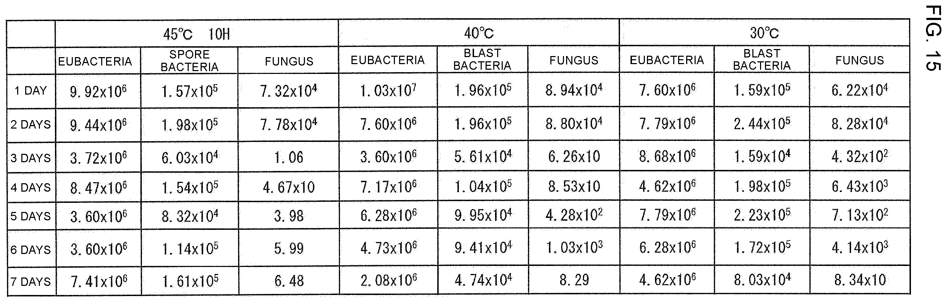

The number of copies per 1 µL by real-time PCR is shown in FIG. 15. FIG. 15 shows the results of measuring changes in the number of fungi caused by heating the culture solution. Although fungi are mixed in the culture solution, the content is about 1/100 of the total number of bacteria. Raising the temperature to 45°C for 10 hours per day can almost eliminate fungi on 3rd day. The number of bacteria and the number of spore bacteria are not reduced by this operation.

### (Oxygen supply capacity by magnetic stirrer)

3 g of Bacillus fermented powder was suspended in 500 ml of water in a cylindrical glass container having a diameter of 9 cm, and stirred and cultured at 40°C using a stirring bar having a length of 2.5 cm, and the oxygen saturation with a culture solution not stirred was compared.

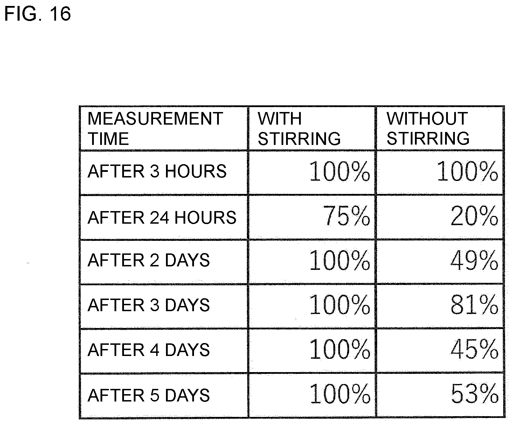

FIG. 16 shows the measurement results of the oxygen saturation. Oxygen having a degree of saturation of 70% or more sufficient for aerobic culture can be supplied to the culture solution by stirring the culture solution with a magnetic stirrer.

### [Other embodiments]

(1) In the above embodiment, the planar heater is wound around the outer periphery of the culture tank unit 10 as the heater unit 30 in order to heat the culture solution to a predetermined temperature, but the culture solution may be heated from the lower portion of the culture tank unit 10 using a hot plate or the like as the top plate 22 of the stirring unit main body 21.
(2) In the above embodiment, the magnetic stirrer is used for stirring and supplying oxygen to the culture solution, but a small air pump or a circulation pump having a shape that facilitates cleaning and disinfection may be used instead of the magnetic stirrer to stir and supply oxygen to the culture solution. However, it is necessary to consider suppression and removal of biofilms in which pathogenic bacteria such as legionella easily propagate.
(3) In the above embodiment, stainless steel is used as the material of the culture tank unit 10, but ceramics, heat resistance/chemical resistance plastic, or the like may be used.
(4) In the above embodiment, as the stirring unit 20, the magnet is rotated by the motor, and the stirrer in the culture tank unit 10 is rotated using the generated magnetic field to stir the culture solution, but instead of the motor, the stirrer may be rotated using the magnetic field generated by applying electricity to the coil.
(5) In the above embodiment, the operation switching of the stirring unit 20 is performed by the power switch provided in the stirring unit main body 21, but the control of the operation switching of the stirring unit may be performed by the control unit 40.

### REFERENCE SIGNS LIST

- 1: Culture solution generator
- 10: Culture tank unit
- 20: Stirring unit
- 21: Stirring unit main body
- 22: Top plate
- 30: Heater unit
- 40: Control unit
- 41: CPU
- 42: ROM
- 43: RAM
- 44: I/O

## Claims

1. A culture solution for promoting immune improvement and normal immune formation, the culture solution obtained by suspending 300 g or more of Bacillus fermented powder in 150 liters of water, maintaining a temperature of 30 to 45°C, and supplying oxygen with an oxygen saturation of 70% or more for fermentation.

2. The culture solution for promoting immune improvement and normal immune formation according to claim 1, the culture solution comprising:
a bacterial concentration of 1 × 10⁹ copies/ml or more in real-time PCR;
180 or more types of bacteria by NSG analysis of a base length of V3 to V4 region 430bp of 16S rDNA; and
Ectobacillus funiculus as a dominant bacterium.

3. A culture solution generator for generating a culture solution for promoting immune improvement and normal immune formation according to claim 1 or 2, the culture solution generator comprising:
a heater unit configured to heat the culture solution;
a stirring unit configured to stir the culture solution; and
a control unit configured to increase a temperature of the culture solution by the heater unit to maintain at a desired temperature.
